# EUROPEAN PATENT SPECIFICATION

(11) **EP 4 375 653 B1**
(45) Date of publication and mention of the grant of the patent: **07.10.2026**
(21) Application number: 22942946.9
(22) Date of filing: 08.11.2022
(51) Int. Cl.: G01N 27/04, G01N 33/24, G01S 13/88, G01N 33/42

(54) **HIGH-PRECISION CONTINUOUS RAPID SUBGRADE MOISTURE MEASUREMENT METHOD AND SYSTEM WITHOUT DAMAGE TO PAVEMENT**
HOCHPRÄZISE, KONTINUIERLICHE UND SCHNELLE MESSUNG DER FEUCHTE DES UNTERBAUS OHNE BESCHÄDIGUNG DES STRASSENBELAGS
PROCÉDÉ ET SYSTÈME DE MESURE D'HUMIDITÉ DE FONDATIONS RAPIDE, CONTINUE ET DE HAUTE PRÉCISION SANS ENDOMMAGEMENT DE CHAUSSÉE

(30) Priority: 26.09.2022 CN 202211175951
(43) Date of publication of application: 29.05.2024
(73) Proprietor: CCCC Second Highway Consultants Co., Ltd., Wuhan, Hubei 430056 (CN)
(72) Inventor: FU, Wei, Wuhan, Hubei 430056 (CN); WANG, Yun, Wuhan, Hubei 430056 (CN); HE, Bin, Wuhan, Hubei 430056 (CN); ZHANG, Chen, Wuhan, Hubei 430056 (CN); LIU, Shuai, Wuhan, Hubei 430056 (CN); ZHU, Kun, Wuhan, Hubei 430056 (CN); RUAN, Yanbin, Wuhan, Hubei 430056 (CN); ZHANG, Jing, Wuhan, Hubei 430056 (CN); LIU, Xing, Wuhan, Hubei 430056 (CN); GU, Lizhou, Wuhan, Hubei 430056 (CN); ZHANG, Hang, Wuhan, Hubei 430056 (CN)
(74) Representative: Gille Hrabal Partnerschaftsgesellschaft mbB Patentanwälte
(86) International application number: PCT/CN2022/130587
(87) International publication number: WO 2024/065960

(56) References cited:
- CN-A- 111 398 687
- CN-A- 114 034 740
- US-A1- 2008 042 653
- "Nondestructive Testing of Materials and Structures", vol. 6, 1 January 2011, SPRINGER NETHERLANDS, ISBN: 978-94-007-0722-1, ISSN: 2211-0844, article KAOUANE CAROLE ET AL: "Condition Assessment of Mixed Paved Roads by GPR and CCR: Material Properties", pages: 861 - 867, XP093259130, DOI: 10.1007/978-94-007-0723-8_123
- SHENG ZENG, LI ZHEN-CUN, ZHAO JIAN, WEI HUI,YIN CHANG-BIN, LI JUN: "New technology of rapid,nondestructive and comprehensive testing for operation highway subgrade disease", JOURNAL OF CHANGSHA UNIVERSITY OF SCIENCE & TECHNOLOGY(NATURAL SCIENCE), vol. 10, no. 2, 28 June 2013 (2013-06-28), pages 1 - 8, XP093151621
- JIN-GUO WANG, YU XIAO-MING, XU CHUN-LONG, WEI JUN-BO: "A study of the methods to extract water content of soil roadbed using ground penetrating radar data ", JOURNAL OF NORTHWEST UNIVERSITY(NATURAL SCIENCE EDITION), vol. 40, 25 February 2010 (2010-02-25), pages 61 - 64, XP093151623
- DE PASCALE GREGORY P., POLLARD WAYNE H., WILLIAMS KEVIN K.: "Geophysical mapping of ground ice using a combination of capacitive coupled resistivity and ground‐penetrating radar, Northwest Territories, Canada", JOURNAL OF GEOPHYSICAL RESEARCH, AMERICAN GEOPHYSICAL UNION, US, vol. 113, no. F2, 1 June 2008 (2008-06-01), US , pages F02S90, XP093151616, ISSN: 0148-0227, DOI: 10.1029/2006JF000585
- LIU SIXIN, LU QI, LI HONGQING, WANG YUANXIN: "Estimation of Moisture Content in Railway Subgrade by Ground Penetrating Radar", REMOTE SENSING, MOLECULAR DIVERSITY PRESERVATION INTERNATIONAL (MDPI), CH, vol. 12, no. 18, 8 September 2020 (2020-09-08), CH , pages 2912, XP093151617, ISSN: 2072-4292, DOI: 10.3390/rs12182912
- WALKER JEFFREY P., HOUSER PAUL R.: "Evaluation of the OhmMapper Instrument for Soil Moisture Measurement", SOIL SCIENCE SOCIETY OF AMERICA. JOURNAL, SOIL SCIENCE SOCIETY OF AMERICA, US, vol. 66, no. 3, 1 May 2002 (2002-05-01), US , pages 728 - 734, XP093151619, ISSN: 0361-5995, DOI: 10.2136/sssaj2002.7280

## Description

### Field of the Invention

The present invention relates to the technical field of subgrade humidity detection, in particular to a high-precision continuous and rapid detection method and system for subgrade humidity without damaging a pavement.

### Background of the Invention

The road subgrade is the foundation of the pavement structure. The humidity state (moisture content) of the subgrade is an important indicator of the strength and stability of the subgrade, and is also one of the key indicators for evaluating the operational state of the subgrade, which directly affects the operational safety and service life of the reconstructed road, so mastering the humidity state of the subgrade is very critical for road maintenance and reconstruction and expansion. In existing engineering practices, the moisture content state of the subgrade is mainly tested through destructive methods such as slotting and drilling sampling. Traditional destructive testing has low efficiency and causes great damage to the pavement structure. At the same time, subgrade soil moisture content sampling detection methods such as drying method, resistance method, neutron probe method, γ ray (transmission) method, and time domain reflectometry (TDR) method, have poor single-point representativeness and low testing efficiency.

As related research on geophysical prospecting methods becomes more mature, geophysical methods such as electromagnetic methods, electrical methods and other technologies are increasingly used in the field of road detection, which have significant advantages in work efficiency and spatial resolution, and can provide basic data on the operational state of the subgrade at any time. At present, the related technology of ground penetrating radar used to qualitatively identify subgrade defects is relatively mature, but in terms of quantitative detection, it mainly focuses on surface layer research. There is less research and application on subgrade moisture content, and the accuracy cannot meet the application. Also due to the limited detection accuracy of the electrical method, which is mostly used for qualitative identification, there are still many uncertainties in the quantitative identification of moisture content.

Different from railway subgrades and road subgrades during construction, operating road subgrades are mostly covered by hardened pavements such as asphalt pavement or cement concrete pavement, the existing road subgrade has become a hidden structure, and the overlying pavement structure makes the existing subgrade moisture content testing more complicated. On the one hand, the pavement structural layer affects the propagation of radar waves in the subgrade soil, which in turn affects the testing accuracy and testing depth, and the existing railway subgrade moisture content testing method is not suitable for the subgrade moisture content testing on existing roads with asphalt/cement pavement conditions; and on the other hand, the traditional electrical method of electrode laying is difficult to implement on hardened pavement structures, and the testing efficiency is difficult to meet the non-destructive testing of long-distance road subgrade moisture content. There is an urgent need to develop a testing equipment system and technical method that can achieve non-destructive continuous detection of road subgrade moisture content so as to test the existing subgrade moisture content covered with asphalt pavements, cement pavements, etc..

The publication "Kaouane Carole ET AL: "Condition Assessment of Mixed Paved Roads by GPR and CCR: Material Properties' in "Nondestructive Testing of Materials and Structures", 1 January 2011, DOI: 10.1007/978-94-007-0723-8 123" relates to a subgrade detection method.

### Summary of the Invention

The purpose of the present invention is to overcome the shortcomings of the above background technology and provide a high-precision continuous and rapid detection method and system for subgrade humidity without damaging a pavement. The invention is defined by the appended claims.

In a first aspect, the present invention provides a high-precision continuous and rapid detection method for subgrade humidity without damaging a pavement, comprising the following steps:
S1: obtaining GPR map data and CCR map data of a road site to be detected;
S2: constructing a pavement material dielectric constant-moisture content relationship model, a pavement material resistivity-moisture content relationship model, and a subgrade filling resistivity-moisture content relationship model;
S3: according to the obtained GPR map data, obtaining a pavement layer thickness and a dielectric constant, and according to the obtained pavement layer thickness and the dielectric constant, as well as the pavement material dielectric constant-moisture content relationship model, obtaining a pavement moisture content; and
S4: according to the CCR map data, the pavement thickness and the pavement moisture content, as well as the subgrade filling resistivity-moisture content relationship model, obtaining a subgrade moisture content spatial distribution.

According to the first aspect, in a first possible implementation manner of the first aspect, step S2 specifically comprises the following steps:
S21: according to data, collecting or gathering representative pavement materials and subgrade filling samples to perform dielectric constant and resistivity tests and obtaining test results; and
S22: fitting the test results to obtain the pavement material dielectric constant-moisture content relationship model, the pavement material resistivity-moisture content relationship model, and the subgrade filling resistivity-moisture content relationship model.

According to the first aspect, in a second possible implementation manner of the first aspect, step S3 specifically comprises the following steps:
S31: preprocessing the obtained GPR map data;
S32: identifying the preprocessed GPR map data and obtaining a pavement structure interface of the road to be detected;
S33: selecting propagation times of radar waves reflected from a bottom of the pavement at different data collection positions on the obtained pavement structure interface;
S34: performing inversion calculations according to a plurality of the obtained propagation times, and obtaining the pavement layer thickness and the dielectric constant; and
S35: according to the obtained dielectric constant and the pavement material dielectric constant-moisture content relationship model, obtaining the pavement moisture content.

Step S4 specifically comprises the following steps:
S41: according to the obtained CCR map data, the pavement layer thickness and the pavement moisture content, obtaining a resistivity spatial distribution within a subgrade range; and
S42: according to the obtained resistivity and the subgrade filling resistivity-moisture content relationship model, obtaining the subgrade moisture content spatial distribution.

Step S41 specifically comprises the following steps:
S410: preprocessing the obtained CCR map data, taking the obtained pavement layer thickness and the pavement moisture content as constraints, taking a least squares of the processed CCR map data and forward calculation results of model parameters, and a sum of regularization terms that stabilize inversion results as objective functions, adopting a Gauss-Newton iteration method to solve the solution and obtaining the resistivity spatial distribution within the subgrade range.

In a second aspect, the present invention provides a high-precision continuous and rapid detection system for subgrade humidity without damaging a pavement, comprising:
an imaging data acquisition module, which is configured to obtain GPR map data and CCR map data of a road site to be detected;
a moisture content relationship model construction module, which is configured to construct a pavement material dielectric constant-moisture content relationship model, a pavement material resistivity-moisture content relationship model, and a subgrade filling resistivity-moisture content relationship model;
a pavement moisture content acquisition module, which is connected to the imaging data acquisition module and the moisture content relationship model construction module in a communication manner, and is configured to obtain a pavement layer thickness and a dielectric constant according to the obtained GPR map data, and obtain a pavement moisture content according to the obtained pavement layer thickness and the dielectric constant, as well as the pavement material dielectric constant-moisture content relationship model; and
a subgrade moisture content acquisition module, which is connected to the imaging data acquisition module, the pavement moisture content acquisition module and the moisture content relationship model construction module in a communication manner, and is configured to obtain a subgrade moisture content spatial distribution according to the CCR map data, the pavement thickness and the pavement moisture content, as well as the subgrade filling resistivity-moisture content relationship model.

According to the second aspect, in a first possible implementation manner of the second aspect, the imaging data acquisition module comprises a multi-channel ground penetrating radar device, a capacitively coupled resistivity meter and a traction vehicle, and the traction vehicle is configured to carry the multi-channel ground penetrating radar device and the capacitively coupled resistivity meter to the road to be detected for data collection.

According to the second aspect, in a second possible implementation manner of the second aspect, the moisture content relationship model construction module comprises:
a test result acquisition submodule, which is configured to collect or gather representative pavement materials and subgrade filling samples according to data to perform dielectric constant and resistivity tests and obtain test results; and
a moisture content relationship model acquisition submodule, which is connected to the test result acquisition submodule in a communication manner, and is configured to fit the test results to obtain the pavement material dielectric constant-moisture content relationship model, the pavement material resistivity-moisture content relationship model, and the subgrade filling resistivity-moisture content relationship model.

According to the second aspect, in a third possible implementation manner of the second aspect, the pavement moisture content acquisition module comprises:
a GPR map data preprocessing submodule, which is connected to the imaging data acquisition module in a communication manner, and is configured to preprocess the obtained GPR map data;
a pavement structure interface acquisition submodule, which is connected to the GPR map data preprocessing submodule in a communication manner, and is configured to identify the preprocessed GPR map data and obtain a pavement structure interface of the road to be detected;
a propagation time acquisition submodule, which is connected to the pavement structure interface acquisition submodule in a communication manner, and is configured to select propagation times of radar waves reflected from a bottom of the pavement at different data collection positions on the obtained pavement structure interface;
a pavement parameter acquisition submodule, which is connected to the propagation time acquisition submodule in a communication manner, and is configured to perform inversion calculations according to a plurality of the obtained propagation times, and obtain the pavement layer thickness and the dielectric constant; and
a pavement moisture content acquisition submodule, which is connected to the pavement parameter acquisition submodule and the imaging data acquisition module in a communication manner, and is configured to obtain the pavement moisture content according to the obtained dielectric constant and the pavement material dielectric constant-moisture content relationship model.

According to the second aspect, the subgrade moisture content acquisition module comprises:
an apparent resistivity acquisition submodule, which is connected to the imaging data acquisition module and the pavement parameter acquisition submodule in a communication manner, and is configured to obtain a spatial distribution of resistivity within a subgrade range according to the CCR map data, the pavement layer thickness and the pavement moisture content; and
a submodule moisture content acquisition submodule, which is connected to the apparent resistivity acquisition submodule and the moisture content relationship model construction module in a communication manner, and is configured to obtain the subgrade moisture content spatial distribution according to the obtained resistivity and the subgrade filling resistivity-moisture content relationship model.

The resistivity acquisition submodule is specifically configured to: preprocess the obtained CCR map data, take the obtained pavement layer thickness and the pavement moisture content as constraints, take a least squares of the processed CCR map data and forward calculation results of model parameters, and a sum of regularization terms that stabilize inversion results as objective functions, adopt a Gauss-Newton iteration method to solve the solution and obtain the resistivity spatial distribution within the subgrade range.

Compared with the prior art, the present invention has the following advantages:
The pavement moisture detection method provided by the present application constructs a moisture content relationship model and indirectly obtains the subgrade moisture content of the road through collecting imaging data of the road to be detected, which has the advantages of not damaging the pavement, fast detection, enabling high-resolution continuous detection in space, and high detection accuracy, and provides reliable data support for maintaining the quality of the subgrade.

### Brief Description of the Drawings

Fig. 1 is a flow chart of a high-precision continuous and rapid detection method for subgrade humidity without damaging a pavement in the embodiment of the present invention;
Fig. 2 is a functional module block diagram of a high-precision continuous and rapid detection system for subgrade humidity without damaging a pavement in the embodiment of the present invention;
Fig. 3 is another flow chart of a high-precision continuous and rapid detection method for subgrade humidity without damaging a pavement in the embodiment of the present invention;
Fig. 4 is a layout diagram of an imaging data acquisition module of a pavement moisture detection system in the embodiment of the present invention;
Fig. 5 is a radar image obtained by a ground penetrating radar in the embodiment of the present invention;
Fig. 6 is a resistivity map obtained by a galvanic coupling resistivity system in the embodiment of the present invention;
Fig. 7 is a pavement layer thickness quantitatively identified by a ground penetrating radar in the embodiment of the present invention;
Fig. 8 is a resistivity distribution obtained by a method of the present invention in the embodiment of the present invention;
Fig. 9 is a subgrade moisture content distribution obtained by a method of the present invention in the embodiment of the present invention.

In the figures: 1-traction vehicle; 2-ground penetrating radar device (GPR); 3-capacitively coupled resistivity meter (CCR); 4-road subgrade pavement structure; 5-main vehicle; 6-trailer one; 7-trailer two; 8-traction rope; 9-radar host; 10-ground couple antenna one; 11-ground couple antenna two; 12-portable computer; 13-power supply; 14-radar wave; 15-receiving electrode; 16-transmitting electrode; 17-cable; 18-insulating rope; 19-galvanic instrument host; 20-display tablet computer; 21-current; 22-pavement; 23-subgrade; 100-imaging data acquisition module; 200-moisture content relationship model acquisition module; 300-pavement moisture content acquisition module; 400-subgrade moisture content acquisition module.

### Detailed Description of the Embodiments

Reference will now be made in detail to the specific embodiments of the present invention, and the embodiments are illustrated in the accompanying drawings. Although the present invention will be described in connection with the specific embodiments, it will be understood that there is no intention to limit the present invention to the embodiments.

In order to make those skilled in the art better understand the present invention, the present invention will be further described below in detail with reference to the drawings in combination with the embodiments.

Note: The embodiment to be introduced next is only a specific embodiment and is not intended to limit the embodiments of the present invention to the following specific steps, values, conditions, data, sequences, etc. Those skilled in the art can apply the concepts of the present invention through reading this specification to construct more embodiments not mentioned in this specification.

The road subgrade is the foundation of the pavement structure, and the subgrade humidity is an important indicator of the strength and stability of the subgrade. Therefore, mastering the subgrade humidity is very critical for road maintenance and expansion. In existing engineering practice, the subgrade humidity is mainly measured through methods such as slotting and drilling sampling, but this has the disadvantages of low detection efficiency and great damage to the pavement structure. The existing non-destructive testing methods, such as an electromagnetic methods and an electrical method, mainly focus on the study of pavement layers, and there are still many uncertainties in the quantitative identification of moisture content; the existing railway subgrade moisture content testing method is not suitable for testing the subgrade moisture content of existing roads with asphalt or cement pavement; and the traditional electrical method is difficult to implement and the testing efficiency is low.

In view of the above, the present application provides a high-precision continuous and rapid detection method for subgrade humidity without damaging a pavement, which effectively solves the technical problems of low detection efficiency, low detection accuracy, and damage to the pavement structure in the existing high-precision continuous and rapid detection method for subgrade humidity without damaging the pavement.

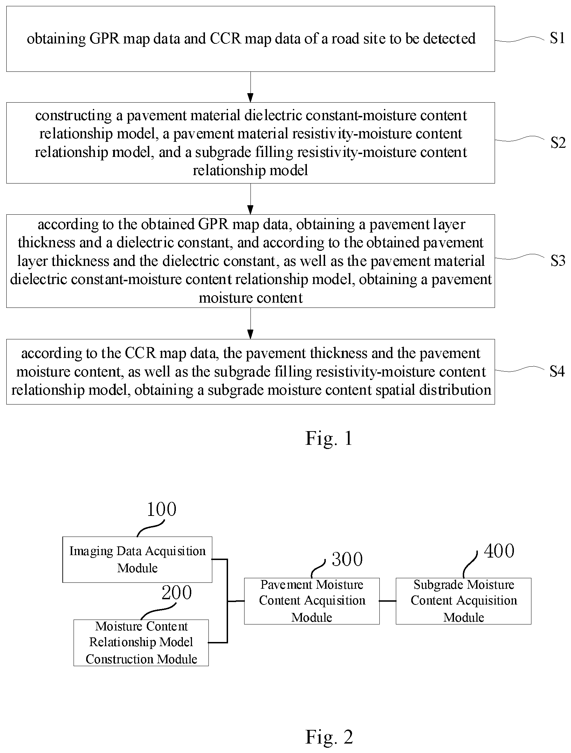

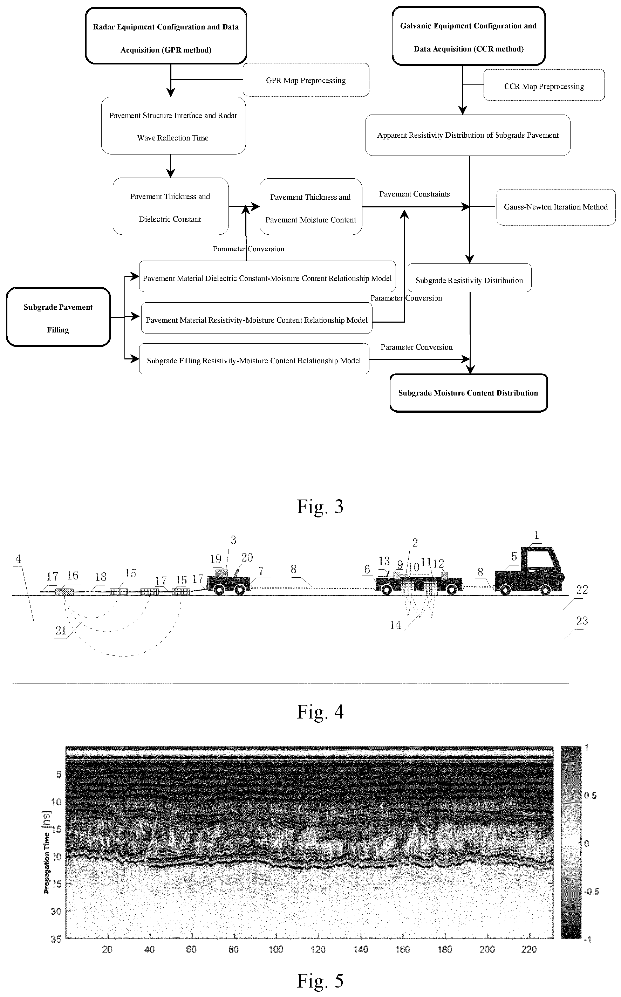

Please refer to Fig. 1 and Fig. 3, the present invention provides a high-precision continuous and rapid detection method for subgrade humidity without damaging a pavement, which comprises the following steps:
S1: obtaining GPR map data and CCR map data of a road site to be detected at the road site to be detected;
S2: constructing a pavement material dielectric constant-moisture content relationship model, a pavement material resistivity-moisture content relationship model, and a subgrade filling resistivity-moisture content relationship model;
S3: according to the obtained GPR map data, obtaining a pavement layer thickness and a dielectric constant, and according to the obtained pavement layer thickness and the dielectric constant, as well as the pavement material dielectric constant-moisture content relationship model, obtaining a pavement moisture content; and
S4: according to the CCR map data, the pavement thickness and the pavement moisture content, as well as the subgrade filling resistivity-moisture content relationship model, obtaining a subgrade moisture content spatial distribution.

The pavement moisture detection method provided by the present application constructs a moisture content relationship model and indirectly obtains the subgrade moisture content of the road through collecting imaging data of the road to be detected, which has the advantages of not damaging the pavement, fast detection, enabling high-resolution continuous detection in space, and high detection accuracy, and provides reliable data support for maintaining the quality of the subgrade.

The high-precision continuous and rapid detection method and system for subgrade humidity without damaging the pavement provided by the present invention have the following beneficial effects compared with the prior art:
(1) In terms of technical reliability, compared with the existing slot sampling testing technology, the slot sampling testing can only detect the moisture content of a single point on the road subgrade pavement structure each time, while the present application can realize linear long-distance continuous testing on the road subgrade pavement structure and obtain the high-density continuous spatial distribution of the subgrade moisture content at different subgrade depths in the direction of survey line. Compared with the single ground penetrating radar method or resistivity method, the ground penetrating radar has good identification accuracy for the pavement structure, but has limited identification information for the subgrade structure below the pavement, and the resistivity method can identify the entire subgrade pavement, but has limited identification accuracy. The present invention combines the advantages of the ground penetrating radar and the resistivity method, uses the ground penetrating radar to identify the structure and moisture content of the pavement, and constrains the resistivity method to identify the subgrade moisture content, which can significantly improve the identification accuracy of the subgrade moisture content. The absolute error of detecting the subgrade moisture content by the method of the present invention is less than 3%. The specific verification process can be found in the embodiments.
(2) In terms of testing efficiency, compared with the existing slot testing, the existing road subgrade pavement structure can only test 2-3 points per day for slot testing of the subgrade moisture content, while the non-destructive testing technology of the present invention can achieve continuous testing of 5-10km per day, and the testing efficiency is improved by an order of magnitude. Compared with the existing traditional electrical methods, such as the high-density resistivity method, the traditional electrical method typically requires electrodes to be inserted into the ground or buried on the pavement, electrode placement requires a significant amount of time, and typically only 2-3 road sections of 100-300m can be tested per day, while the GPR and CCR devices of the present invention can achieve ground coupling contact on asphalt or cement hardened pavements, and are continuously tested by manual or vehicle traction and dragging, without the need for specialized electrode laying and arrangement, greatly improving the testing efficiency.
(3) In terms of economic and environmental protection, compared with the widely used slot testing, the slot testing requires crushing and excavation of the upper structure of the pavement 4 and roadbed 5 at the testing point, large equipment such as excavators are usually used, and the trenches after excavation testing need to be backfilled and repaired. However, it is usually difficult to restore the original subgrade structure, which can easily lead to local secondary pavement diseases. Therefore, the cost of testing a single point is high, time-consuming, labor-intensive and not environmentally friendly. The present invention realizes non-destructive testing through GPR and CCR, has no damage to the road subgrade pavement structure, has zero emissions, is clean and efficient, and reduces the testing cost by at least 60%.

As mentioned above, the humidity can also be referred to as moisture content.

In one embodiment, before the S1, the following steps are further comprised.

S0: According to the structural form of the road to be detected, type of subgrade filling material, environmental conditions, etc., an appropriate multi-channel ground penetrating radar device (GPR), a capacitively coupled resistivity meter (CCR) and a traction vehicle are selected to construct a high-precision continuous and rapid detection system for subgrade humidity without damaging a pavement, and the parameter configuration such as GPR test frequency and CCR electrode distance are determined.

In one embodiment, the S1 specifically comprises the following steps:
at the road site to be detected, the multi-channel ground penetrating radar test device and capacitively coupled resistivity meter are connected respectively, parameter configurations such as GPR test frequency and CCR electrode distance are adjusted, and the traction vehicle is equipped with GPR and CCR and runs the test at a certain driving speed on the subgrade payment structure of the road to be detected, and collects and obtains the GPR map data and CCR map data of the road section to be detected.

In one embodiment, the certain driving speed is implemented as a preset speed.

In one embodiment, the S2 specifically comprises the following steps.
S21: according to data, collecting or gathering representative pavement materials and subgrade filling samples to perform dielectric constant and resistivity tests and obtaining test results; and
S22: fitting the test results to obtain the pavement material dielectric constant-moisture content relationship model, the pavement material resistivity-moisture content relationship model, and the subgrade filling resistivity-moisture content relationship model.

In a more specific embodiment, the S2 is specifically implemented to collect 1-2 sets of samples for each pavement material and subgrade filling. The pavement material needs to test the dielectric constant and resistivity under 3-5 different moisture content conditions, and the subgrade filling needs to test the resistivity under 3-5 different moisture content conditions, so as to obtain the pavement material dielectric constant-moisture content relationship model, the pavement material resistivity-moisture content relationship model, and the subgrade filling resistivity-moisture content relationship mode by fitting.

In one embodiment, the relationship model between the dielectric constant and the moisture content in the S2 adopts an empirical formula (S2-0-1) to obtain specific model parameters C₀₀, C₀₁, C₀₂, and C₀₃ through indoor tests or similar engineering surveys. ${w}_{v} = {C}_{00} + {C}_{01} {ε}_{b} + {C}_{02} {{ε}_{b}}^{2} + {C}_{03} {{ε}_{b}}^{3}$ Formula (S2-0-1) wherein *ε_{b}* is the dielectric constant; *wᵥ* is the volume moisture content; *C₀₀, C₀₁, C₀₂, C₀₃* are empirical constants.

As a preferred technical solution of the present invention, the resistivity-moisture content relationship model in the step S2 can adopt an Archie formula for asphalt concrete, cement-stabilized macadam, and coarse-grained fillings such as gravel and sandy soil: $ρ = {ρ}_{w} {{n}_{s}}^{- x} {{S}_{w}}^{- y}$ Formula (S2-0-2) wherein ρ is the resistivity of filling, *ρ_{w}* is the resistivity of pore water; *nₛ* is the porosity of soil body; *S_{w}* is the pore water saturation of subgrade soil, *S_{w}=wᵥ*/*nₛ;* x is an index related to the cementation degree of the soil body; and y is the saturation exponent. For coarse-grained soil subgrade fillings such as gravel soil, gravel, and sandy soil, the value range of x is 1.0 to 5.0, and the value range of y is usually 1.5 to 2.5.

For the fine-grained soil fillings such as cohesive soil and lime-improved soil, the empirical formula is: $ρ = \frac{{ρ}_{w}}{{c}_{c} {{w}_{v}}^{p}}$ Formula (S2-0-3) *c_{c}* and p are fitting parameters related to the size of soil particles, and are related to the volume percentage *θ_{c}* of clay in the filling, *c_{c}* = *x*₁*θ_{c}*^{*y*1} and *p = x₂θ_{c}^{y2};* when *θ_{c}* ≥5%, *x*₁=0.6, y1=0.55, *x*₂=0.92 and y1=0.2; and when *θ_{c}* <5%, *c_{c}=1.45* and p=1.25.

In one embodiment, the S3 specifically comprises the following steps.
S31: preprocessing the obtained GPR map data;
S32: identifying the preprocessed GPR map data and obtaining a pavement structure interface of the road to be detected;
S33: selecting propagation times of radar waves reflected from a bottom of the pavement at different data collection positions on the obtained pavement structure interface;
S34: performing inversion calculations according to a plurality of the obtained propagation times, and obtaining the pavement layer thickness and the dielectric constant; and
S35: according to the obtained dielectric constant and the pavement material dielectric constant-moisture content relationship model, obtaining the pavement moisture content.

In one embodiment, the S34 specifically comprises the following steps.

As a preferred technical solution of the present invention, the GPR map data preprocessing in the S3 first uses the demeaning algorithm (DEWOW) for low-frequency noise interference, and then selects the propagation time ti corresponding to the bottom reflection signal of the pavement 22 at different collection locations.

The calculation of pavement thickness and dielectric constant in the S3 is based on the principle that the reflected wave propagation time ti is a functional relationship (S3-0-1) of three parameters: the reflection depth (i.e., the thickness of the pavement layer) hi, the dielectric constant εbi, and the reflection surface inclination angle αi of the reflected wave. Through combining a plurality of antennas of the multi-channel ground penetrating radar collected, each data collection point can obtain the at least three propagation times corresponding to different antenna spacings. Through using the formula (S3-0-1), the three sets of data can be combined to solve for three unknowns, namely the pavement thickness hi, the dielectric constant εbi and the reflection surface inclination angle αi of the reflected wave. ${t}_{mod} \left({d}_{i}; {h}_{i}; {ε}_{bi}; {α}_{i}\right) = \frac{{ε}_{bi}}{{c}_{0}} cos {α}_{i} \sqrt{4 {h}_{i}^{2} + {d}_{i}^{2}}$ Formula (S3-0-1)

When calculating the specific operation of the pavement thickness and the dielectric constant, according to n observation times *t_{obs}* (observation time) data collected by the multi-channel ground penetrating radar at different test locations, the formula (S3-0-1) is adopted to simulate and calculate n corresponding radar wave propagation times t_{mod} (simulation time), and all t_{obs} and t_{mod} are combined to construct an objective function formula (S3-0-2), and through optimization algorithms such as Gaussian iteration, the parameter combination (hi, ε_{bi}, αi) that minimizes the parameter difference between the observed value and the simulated value is obtained, that is, the pavement thickness hi and the dielectric constant ε_{bi} are obtained at different test positions. $Γ \left({h}_{i}, {ε}_{bi}, {α}_{i}\right) = {\sum }_{i}^{n} {\left({t}_{obs}\left({d}_{i}\right)-{t}_{mod}\left({d}_{i}; {h}_{i}; {ε}_{bi}; {α}_{i}\right)\right)}^{2}$ Formula (S3-0-2) wherein *t_{obs}* is the propagation time of the electromagnetic wave signal collected by the on-site radar from the transmitting end to the receiving end after being reflected from the bottom of the pavement, *t_{mod}* is the electromagnetic wave propagation time obtained based on the parameter simulation, dᵢ is the distance between the antenna transmitting and receiving ends, hᵢ is the pavement thickness, αᵢ is the angle between the radar wave reflection surface and the horizontal plane, ε_{bi} is the dielectric constant of the filling, c0 is a constant, i is the data point number collected by the radar, n is the total number of data collection points.

According to the invention, step S4 specifically comprises the following steps:
S41: according to the obtained CCR map data, the pavement layer thickness and the pavement moisture content, obtaining a resistivity spatial distribution within a subgrade range; and
S42: according to the obtained resistivity and the subgrade filling resistivity-moisture content relationship model, obtaining the subgrade moisture content spatial distribution.

According to the invention, step S41 specifically comprises the following step:
S410: preprocessing the obtained CCR map data, taking the obtained pavement layer thickness and the pavement moisture content as constraints, taking a least squares of the processed CCR map data and forward calculation results of model parameters, and a sum of regularization terms that stabilize inversion results as objective functions, adopting a Gauss-Newton iteration method to solve the solution and obtaining the resistivity spatial distribution within the subgrade range.

In one embodiment, the S410 is specifically implemented as:
in one embodiment, the CCR spectrum data preprocessing in the S4 first preprocesses the measured apparent resistivity data to eliminate outliers in the apparent resistivity data, then uses the apparent resistivity data as input to the inversion algorithm, and uses the pavement thickness and moisture content obtained by the ground penetrating radar as a priori constraint information for inversion. Taking the pavement moisture content as a constraint, it is necessary to first adopt the pavement material resistivity-moisture content relationship model obtained in the S2 to convert the moisture content calculated by the ground penetrating radar into the pavement resistivity.

As a preferred technical solution of the present invention, the resistivity calculation method in the S4 uses the least squares of the CCR observation data and the forward calculation results of the model parameters as a fitting term: ${Φ}_{ρ} = {\left(ρ-F\left(m\right)\right)}^{T} {W}_{ρ}^{T} {W}_{ρ} \left(ρ-F\left(m\right)\right)$ Formula (S4-0-1) wherein ρ is the measured apparent resistivity data (calculated by the formula S4-0-2), m is the resistivity model, a resistivity spatial distribution matrix composed of simulated values of resistivity at different grid points, F (m) is the forward simulation of resistivity model m, and Wρ is the data weight matrix. If it is assumed that the observed data errors are uncorrelated and the forward simulation numerical errors are ignored, the data weight matrix is a diagonal matrix equal to the standard deviation of the measured data. $ρ = KV / I$ Formula (S4-0-2) $K = \frac{lπ}{ln \left\{{\left[\frac{{b}^{2}}{{b}^{2} - 1}\right]}^{2 b}{\left[\frac{{b}^{2} + 2 b}{{\left(b+1\right)}^{2}}\right]}^{b + 2}{\left[\frac{{b}^{2} - 2 b}{{\left(b-1\right)}^{2}}\right]}^{b - 2}\right\}}$ Formula (S4-0-3) wherein I is the supply current, V is the measured potential, ρ is the apparent resistivity, K is the device coefficient (calculated by the formula E-0-3), b=2r/l, l is the length of the transmitting and receiving dipoles, and r is the dipole center distance.

In order to make the inversion result stable and unique, a regularization term is added: ${Φ}_{m} = \left‖C\left(m-{m}_{ref}\right)\right‖$ Formula (S4-0-4) wherein *m_{ref}* is the initial resistivity model. If the initial resistivity model does not contain known information, matrix C is usually a smooth matrix to obtain a model that reflects smooth changes in resistivity. In order to take the radar results as a priori information to constrain the inversion and achieve the purpose of independently controlling the inversion model boundaries and model unit characteristics, the matrix C is set as: $C = diag \left({w}_{i}^{s}\right) {C}_{1} diag \left({w}_{j}^{n}\right)$ Formula (S4-0-5) wherein *C*₁ is the difference matrix, *diag*(*wⱼⁿ*) is the diagonal weight matrix that controls n grid cells in the model respectively, *diag(wᵢ^{s})* is the diagonal weight matrix that controls s individual structures in the model respectively, *wᵢ^{s}* and *wⱼⁿ* are the diagonal weight values, i=1... s, j=1...n, at the interface between subgrade soil and pavement or bedrock, ${w}_{i}^{s} = 0$ is set.

The final inversion objective function is: $Φ = {Φ}_{ρ} + λ {Φ}_{m}$ Formula (S4-0-6) wherein *Φ_{ρ}* is the observation data fitting term, *Φₘ* is the regularization term, and *λ* is the weight coefficient that controls the observation data fitting term and regularization term.

The inversion process is to find the parameter value that minimizes the objective function. The Gauss-Newton iteration method is used to solve the solution, and the model parameter change matrix for each iteration is obtained through the following equation: $\begin{matrix}\left({J}_{k}^{T}{W}_{ρ}^{T}{W}_{ρ}{J}_{k}+{λC}^{T}C\right) V m = {J}_{k}^{T} {W}_{ρ}^{T} {W}_{ρ} \left(ρ-F\left({m}_{k}\right)\right) - {λC}^{T} C \left({m}_{k}-{m}_{ref}\right) \\ {m}_{k + 1} = {m}_{k} + V m\end{matrix}$ Formula (S4-0-7) wherein J is the Jacobian matrix *J_{i,j}* at the k-th iteration, and *J*_{i,j} = *∂ρᵢ*|*∂mⱼ*; *mₖ* is the model parameter value at the k-th iteration; and Δ*m* is the parameter change at the k-th iteration. *λ* is the weight coefficient, and the value of *λ* constantly changes during the process of iteration, a larger value of *λ* is adopted at the beginning of the iteration and a smaller value of *λ* near convergence is more conducive to the convergence of the inversion. Through iterative solution, the resistivity model m is finally obtained, that is, the resistivity distribution of the subgrade is solved.

Based on the same inventive concept, please refer to Fig. 2, the present invention provides a high-precision continuous and rapid detection system for subgrade humidity without damaging a pavement, which comprises:
an imaging data acquisition module 100, which is configured to obtain GPR map data and CCR map data of a road site to be detected;
a moisture content relationship model construction module 200, which is configured to construct a pavement material dielectric constant-moisture content relationship model, a pavement material resistivity-moisture content relationship model, and a subgrade filling resistivity-moisture content relationship model;
a pavement moisture content acquisition module 300, which is connected to the imaging data acquisition module 100 and the moisture content relationship model construction module 200 in a communication manner, and is configured to obtain a pavement layer thickness and a dielectric constant according to the obtained GPR map data, and obtain a pavement moisture content according to the obtained pavement layer thickness and the dielectric constant, as well as the pavement material dielectric constant-moisture content relationship model; and
a subgrade moisture content acquisition module 400, which is connected to the imaging data acquisition module 100, the pavement moisture content acquisition module 200 and the moisture content relationship model construction module 300 in a communication manner, and is configured to obtain a subgrade moisture content spatial distribution according to the CCR map data, the pavement thickness and the pavement moisture content, as well as the subgrade filling resistivity-moisture content relationship model.

In one embodiment, the imaging data acquisition module comprises a multi-channel ground penetrating radar device, a capacitively coupled resistivity meter and a traction vehicle, and please refer to Fig. 4, the traction vehicle is configured to carry the multi-channel ground penetrating radar device and the capacitively coupled resistivity meter to the road to be detected for data collection.

The ground penetrating radar device 2 (GPR) comprises a radar host 7, more than 2 ground couple antennas (comprising ground couple antenna one 8 and ground couple antenna two 9), a portable computer 10, and a power supply 11. The ground couple antennas are connected to the GPR host through signal lines respectively, the power supply is connected to the GPR host through a power cord, the portable computer is connected to the GPR host through a signal line, the ground couple antennas are placed on the lower part of the trailer body, and the GPR host, power supply, and portable computer are placed on the upper part of the trailer. During the test, the traction vehicle is equipped with a channel ground penetrating radar device and travels on the road section to be tested, transmitting instructions and receiving instructions are sent through the GPR host controlled by the portable computer, the transmitting antenna transmits radar waves to the road subgrade pavement structure according to the transmitting instructions of the GPR host, the receiving antenna collects radar waves reflected from the road subgrade pavement structure according to the receiving instructions, and the received reflected radar wave signals are displayed and saved in the portable computer.

The capacitively coupled resistivity meter (CCR) comprises one transmitting electrode, more than two receiving electrodes, cables, a host and a display tablet computer. The transmitting electrode are connected to a cable at both ends as a transmitting dipole, the receiving electrode is connected to a cable at both ends as a receiving dipole, the transmitting dipole and the receiving dipole are connected by an insulating rope with adjustable length, the receiving dipole is connected to the host through an optical cable, the transmitting dipole is not connected to the host, the host and the display tablet computer are connected through a Bluetooth, and the display tablet computer has a built-in GPS module that records the location coordinates during data collection in real time. During the test, the power switches of all the receiving antennas and transmitting antennas are turned on, and the capacitively coupled resistivity meter is dragged by the trailer two of the traction vehicle on the road section to be tested, the transmitting dipole supplies current 21 to the road subgrade pavement structure, the receiving dipole collects the output current passing through the road subgrade pavement structure, the display tablet computer controls the host to collect the current information data of the receiving dipole, and the current information data is displayed and saved in the display tablet computer.

The traction vehicle is composed of a main vehicle and two trailers. The main vehicle and trailer one are connected through a 2-3m long traction rope, and trailer one and trailer two are connected through a 10-20m long traction rope, the ground couple antennas of the multi-channel ground penetrating radar device are placed in the lower part of the trailer one, and the radar host, portable computer, and power supply are connected through power cords or signal lines and placed in the upper part of the trailer one; and the host and the tablet computer of the capacitively coupled resistivity meter are placed on the trailer two, and the transmitting electrode and the receiving electrode are connected in series and connected to the rear of the trailer two through cables and insulating ropes. During the test, the traction vehicle is equipped with GPR and CCR and runs the test on the road subgrade pavement structure at a certain driving speed, the GPR host controls the transmitting antenna to transmit radar waves to the road subgrade pavement structure, the receiving antenna collects the radar wave signal reflected from the road subgrade pavement structure, the radar wave map is saved to the portable computer, the CCR transmitting dipole supplies current to the road subgrade pavement structure, the receiving dipole collects the output current passing through the road subgrade pavement structure, and the CCR host collects the current information data of the receiving dipole and saves the current information data to the tablet computer; and the collected GPR and CCR map data are imported into the data analysis algorithm for processing so as to obtain the subgrade moisture content distribution.

The main vehicle can be a general commercial off-road vehicle or a minivan. The traction vehicle has a body made of plastic and is equipped with 4 high-strength rubber wheels. The body of the traction vehicle is 1.5-2.0m long and 0.6-1.0m wide. The cart has a load capacity of more than 100kg and can safely and stably carry GPR and CCR components. The traction rope has a tensile bearing capacity of more than 5kN.

The GPR host adopts conventional commercial multi-channel ground penetrating radar, which can control and collect data from a plurality of antenna channels, the time window range of data collection is continuously adjustable from 20ns to 200s, with 512 to 32767 sampling points per scan, the power consumption of the whole host is less than 6 W, and the minimum sampling interval is less than 2ps.

The ground couple antenna is a ground coupled antenna with a fixed distance between the transmitting end and the receiving end, and there are more than 2 ground couple antennas; and the antenna frequency is generally 200-1200MHz, for roads with a pavement thickness of about 80cm, such as expressways and first-class highways, it is recommended to choose 600MHz, and for roads with a pavement thickness of about 50cm, such as highways below class 2, it is recommended to choose 800MHz.

The portable computer is a 3-proof rugged portable computer, with a drop resistance height of not less than 1m, a display of more than 10 inches (25 cm); highly readable in sunlight, a memory of more than 8G and a hard disk storage of more than 256G, and is equipped with a Windows 7 and above operating system and a radar data collection and processing software that is compatible with the host, which can achieve interactive control and data storage of the host.

The power supply is a rechargeable mobile power supply with an output power of 220v and a power supply capacity of more than 10A.

The working frequency of the transmitting electrode is about 16.5KHz, the maximum output power is 2 W, the output voltage is less than 1000Vrms, and the output current is 0.125-16mA.

The input voltage of the receiving electrode is 0-2Vrms.

The host is a capacitively coupled resistivity meter host, with a maximum acquisition time of 2 scans per second, and can work continuously for more than 24 hours.

The cable is a dipole coaxial cable with an outer diameter of 1.5-3.0 cm, and comprises a coaxial insulation layer, a coaxial conductive layer, and a conductive core from the outside to the inside. The length of each cable section is 2.5m or 5.0m. When the test depth is 2m (within the range of roadbed 5), 2.5m cable is usually used, and when the test depth is 4m (within the range of roadbed 5 and the upper part of embankment 6), 5m cable is used.

The tablet computer is a tablet computer with Bluetooth communication function, a memory of more than 8G, and a hard disk storage of more than 256G, and is equipped with a resistivity imaging data acquisition and processing software that is compatible with the host, which can achieve interactive control and data storage of the host.

The insulating rope has a tensile bearing capacity of more than 2kN and a length of 2.5-10.0m.

The road subgrade pavement structure 1 comprises an embankment, roadbed, and pavement from bottom to top. The subgrade is filled with crushed stone soil, sandy gravel soil, or cohesive soil, and the pavement is filled in layers by graded crushed rock, cement-stabilized macadam, cement concrete, and asphalt concrete, etc.

The driving speed of the traction vehicle is generally 3.0-10.0km/h.

In one embodiment, the moisture content relationship model construction module comprises:
a test result acquisition submodule, which is configured to collect or gather representative pavement materials and subgrade filling samples according to data to perform dielectric constant and resistivity tests and obtain test results; and
a moisture content relationship model acquisition submodule, which is connrcted to the test result acquisition submodule in a communication manner, and is configured to fit the test results to obtain the pavement material dielectric constant-moisture content relationship model, the pavement material resistivity-moisture content relationship model, and the subgrade filling resistivity-moisture content relationship model.

In one embodiment, the pavement moisture content acquisition module comprises:
a GPR map data preprocessing submodule, which is connected to the imaging data acquisition module in a communication manner, and is configured to preprocess the obtained GPR map data;
a pavement structure interface acquisition submodule, which is connected to the GPR map data preprocessing submodule in a communication manner, and is configured to identify the preprocessed GPR map data and obtain a pavement structure interface of the road to be detected;
a propagation time acquisition submodule, which is connected to the pavement structure interface acquisition submodule in a communication manner, and is configured to select propagation times of radar waves reflected from a bottom of the pavement at different data collection positions on the obtained pavement structure interface;
a pavement parameter acquisition submodule, which is connected to the propagation time acquisition submodule in a communication manner, and is configured to perform inversion calculations according to a plurality of the obtained propagation times, and obtain the pavement layer thickness and the dielectric constant; and
a pavement moisture content acquisition submodule, which is connected to the pavement parameter acquisition submodule and the imaging data acquisition module in a communication manner, and is configured to obtain the pavement moisture content according to the obtained dielectric constant and the pavement material dielectric constant-moisture content relationship model.

According to the invention, the subgrade moisture content acquisition module comprises:
an apparent resistivity acquisition submodule, which is connected to the imaging data acquisition module and the pavement parameter acquisition submodule in a communication manner, and is configured to obtain an apparent resistivity within a subgrade range according to the CCR map data, the pavement layer thickness and the pavement moisture content; and
a submodule moisture content acquisition submodule, which is connected to the apparent resistivity acquisition submodule and the moisture content relationship model construction module in a communication manner, and is configured to obtain the subgrade moisture content spatial distribution according to the obtained apparent resistivity and the subgrade filling resistivity-moisture content relationship model.

The resistivity acquisition submodule is specifically configured to: preprocess the obtained CCR map data, take the obtained pavement layer thickness and the pavement moisture content as constraints, take a least squares of the processed CCR map data and forward calculation results of model parameters, and a sum of regularization terms that stabilize inversion results as objective functions, adopt a Gauss-Newton iteration method to solve the solution and obtain the resistivity spatial distribution within the subgrade range.

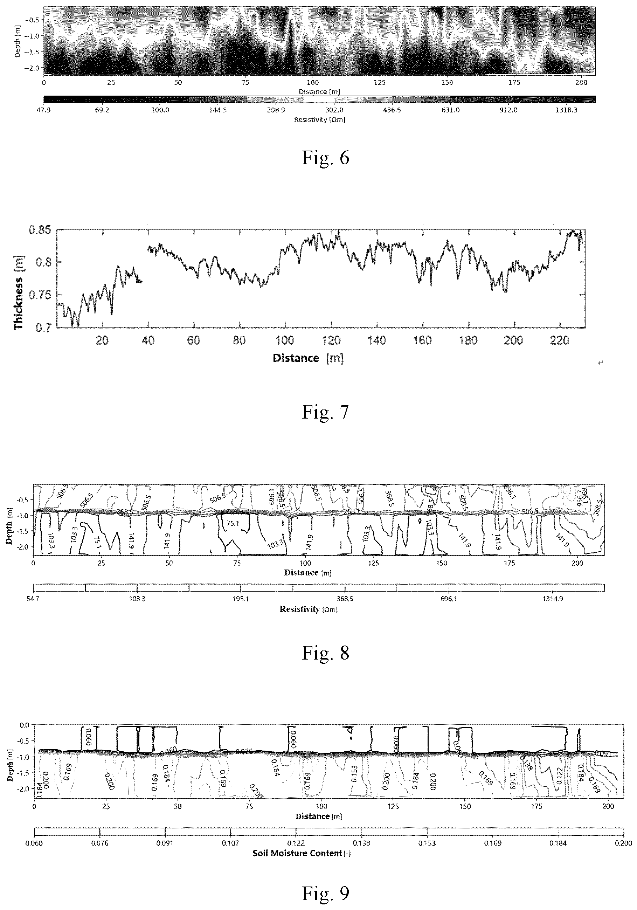

In a specific testing case, an expressway reconstruction and expansion project in South China has two-way 6-lane, the design speed of the entire line is 80km/h, the pavement structure is a 15cm asphalt concrete + 68cm cement-stabilized crushed stone layer, the roadbed filling is fine-grained soil and gravel, and the test road section is a slow lane. The test comprises 6 road sections, each section is 1500m and the total length is 9000m. The test process is as follows:
1. A multi-channel ground penetrating radar device 2 (GPR), a capacitively coupled resistivity meter 3 (CCR), and a traction vehicle are selected to form an imaging data acquisition module of a high-precision continuous and rapid detection system for subgrade humidity without damaging a pavement, and the test is performed on the subgrade 23 and the pavement 22, as shown in Fig. 4. The GPR system is a multi-channel ground penetrating radar HI-MOD, which can control and collect data from a plurality of antenna channels, the time window range of data collection is continuously adjustable from 20ns to 200s, with 512 to 32767 sampling points per scan, two ground couple antennas are configured, and the antenna frequency is 900MHz; and the ground couple antennas are connected to the GPR host through signal lines respectively, the power supply is connected to the GPR host through a power cord, the portable computer is connected to the GPR host through a signal line, and the transmitting antenna and receiving antenna are placed on the fixing frame at the front body of the cart 12, the GPR host and power supply are placed on the fixing frame at the middle body of the cart 12, and the portable computer is placed on the support platform at the rear of the cart 12. The CCR system is the OhmMapper TRN capacitively coupled resistivity meter, which is equipped with one transmitting electrode 16 and four receiving electrodes 15. The working frequency of the transmitting electrode is about 16.5KHz, the output current is 0.125-16mA, and the input voltage of the receiving electrode is 0-2Vrms. The transmitting electrode are connected to a cable at both ends as a transmitting dipole, the receiving electrode is connected to a cable 17 at both ends as a receiving dipole, the transmitting dipole and the receiving dipole are connected by an insulating rope 18 with adjustable length, the receiving dipole is connected to the host through an optical cable, the transmitting dipole is not connected to the host 19, the host 19 and the display tablet computer 20 are connected through a Bluetooth.
2. The test system is configured parameters according to the site conditions, the distance between the ground couple antennas is set to 1.0m, and the distance between the transmitting and receiving dipole moments of the capacitively coupled resistivity meter is 2.5 m. After the parameters are set, the test operator 13 starts the traction vehicle on the road to be detected and drives at a speed of 3.0-5.0km/h, the road is detected through the distributed drag ground penetrating radar device and the capacitively coupled resistivity system through the trailer one and trailer two. The GPR and CCR are tested simultaneously and independently to complete the data collection. Fig. 5 and Fig. 6 show the representative collection results for a test road section one of 200m.
3. The pavement materials are collected, such as 2 sets of samples each for asphalt concrete, cement-stabilized macadam and subgrade filling (comprising fine-grained soil and gravel), the dielectric constant and resistivity of pavement materials need to be tested under 3-5 different moisture content conditions, and the resistivity of the subgrade filling needs to be tested under 5 different moisture content conditions, so as to obtain the dielectric constant-moisture content relationship model and resistivity-moisture content relationship model by fitting. The relationship model between the dielectric constant and moisture content of asphalt concrete and cement-stabilized macadam uses the empirical formula (S2-0-1), C00=-6.216, C01=2.383, C02=-0.0598, C03=0.0006. The resistivity-moisture content relationship model of asphalt concrete, cement-stabilized macadam, and fine-grained soil and gravel adopts an Archie's formula (S2-0-2), the comprehensive parameter values of the pavement material of the asphalt concrete and the cement-stabilized macadam are: ρw is 60Ωm, ns is 0.12, x is 1.5, and y is 1.8. The parameter values of the subgrade material comprising the fine-grained soil and gravel are: ρw is 60Ωm, ns is 0.36, x is 3.4, and y is 2.3.
4. The collected GPR map data is performed preprocessing and the pavement structure interface is identified through the processed imaging map, as shown in Fig. 7. The thickness of the pavement is 0.70-0.85m, the propagation time of the radar waves reflected from the bottom of the pavement is selected at different collection locations. According to n observation times tobs (observation time) data collected by the multi-channel ground penetrating radar at different test locations, the formula (S3-0-1) is adopted to simulate and calculate n corresponding radar wave propagation times tmod (simulation time), and all tobs and tmod are combined to construct an objective function formula (S3-02), and through optimization algorithms such as Gaussian iteration, the parameter combination (hi, ε_{bi}, αᵢ) that minimizes the parameter difference between the observed value and the simulated value is obtained, that is, the pavement thickness hᵢ and dielectric constant ε_{bi} are obtained at different test positions, and then the dielectric constant of the pavement layer is converted into the pavement moisture content through the determined dielectric constant-moisture content relationship model.
5. The collected CCR map data is performed preprocessing, and the pavement thickness and moisture content obtained in the previous step are taken as the constraint equations (S4-0-4, S4-0-5) for resistivity calculation, the least squares of the CCR observation data and forward calculation results of model parameters and the sum of regularization terms that stabilize inversion results are taken as objective functions (S4-0-6), a Gauss-Newton iteration method is adopted to solve the solution (S4-0-7) so as to obtain the resistivity distribution within the subgrade range, and the joint inversion results are shown in Fig. 8.
6. The determined subgrade filling resistivity-moisture content relationship model is adopted to convert the apparent resistivity of the subgrade soil obtained in the previous step into the moisture content. The finally obtained spatial distribution of moisture content of the pavement layer and the upper part of the subgrade (roadbed) is shown in Fig. 9.

Through the above steps, rapid and continuous testing of road subgrade humidity without damaging the pavement can be achieved. In order to verify the accuracy of the method of the present invention in detecting the moisture content of the subgrade, one trench is opened in each detection road section, and the depth of the trench is 30cm below the top surface of the roadbed. The measured moisture content of the subgrade after sampling and drying is compared with the moisture content obtained by the non-destructive testing method of the present invention. The results are shown in Table 1. The absolute error of detecting the moisture content is -2.8% to 2.1%, and the accuracy is higher than that of the existing single resistivity method or radar detection method, which can be seen that the high-precision continuous and rapid detection method for subgrade humidity without damaging the pavement provided by the present application has high accuracy and reliability.

**Table 1 Subgrade Moisture Content Verification and Accuracy Analysis**

| Serial Number | Subgrade Type | Subgrade Soil Type | slotting measured moisture content (%) | Moisture Content Measured by High-precision Continuous and Rapid Detection Method for Subgrade Humidity without Damaging Pavement of Present Invention (%) | Absolute Error (%) |
|---|---|---|---|---|---|
| Verification Point 1 | Soft-Soil Foundation Fill Subgrade | Containing Fine-grained Soil Sand (Medium Sand) | 14.2% | 12.40% | -1.80% |
| Verification Point 2 | Low Filling and Shallow Excavation Fill Subgrade | Containing Fine-grained Soil Sand (Coarse Sand) | 8.9% | 10.10% | 1.20% |
| Verification Point 3 | Excavation Subgrade Low Filling and Shallow Excavation | Containing Fine-grained Soil Sand (Coarse Sand) | 14.3% | 11.50% | -2.80% |
| Verification Point 4 | Adjacent Reservoir Embankment High Fill Subgrade | Containing Fine-grained Soil Sand (Coarse Sand) | 11.6% | 13.70% | 2.10% |
| Verification Point 5 | Fill Subgrade Low Filling and Shallow Excavation | Containing Fine-grained Soil Sand (Medium Sand) | 15.6% | 15.20% | -0.40% |
| Verification Point 6 | Half Filling and Half Digging Fill Subgrade | Containing Fine-grained Soil Sand (Medium Sand) | 12.9% | 14.30% | 1.40% |

Based on the same inventive concept, the embodiment of the present invention provides a computer-readable storage medium, on which a computer program is stored. When the computer program is executed by a processor, all or part of the method steps of the above method are realized.

The present invention implements all or part of the processes in the above method, and can also be completed by instructing relevant hardware through a computer program, the computer program can be stored in a computer-readable storage medium, and when executed by the processor, the computer program can implement the steps of each of the above method embodiments. The computer program comprises computer program code, which can be in the form of source code, object code, executable file or some intermediate. The computer-readable media can comprise any entity or device capable of carrying computer program code, recording media, U disk, mobile hard disk, magnetic disk, optical disk, computer memory, read-only memory (ROM), random access memory (RAM), electrical carrier signal, telecommunication signal and software distribution medium. It should be noted that the contents contained in the computer-readable media can be appropriately increased or decreased according to the requirements of legislation and patent practice in jurisdictions. For example, in some jurisdictions, according to the legislation and the patent practice, the computer-readable media does not comprise electrical carrier signals and telecommunication signals.

Based on the same inventive concept, the embodiment of the present invention also provides an electronic device, comprising a memory and a processor, wherein the memory stores the computer program running on the processor, and the processor implements all or part of the method steps in the embodiments when the computer program is executed.

The processor can be a central processing unit (CPU), or other general-purpose processors, digital signal processor (DSP), application specific integrated circuit (ASIC), field-programmable gate array (FPGA) or other programmable logic devices, discrete gate or transistor logic devices, discrete hardware components, etc. The general-purpose processor can be a microprocessor or any conventional processor, etc. The processor is the control center of the computer device and connects various parts of the whole computer device by means of various interfaces and lines.

The memory can be configured to store computer programs and/or modules, and the processor implements various functions of the computer device through running or executing the computer programs and/or modules stored in the memory, and through calling the data stored in the memory. The memory may mainly comprise a storage program area and a storage data area, wherein the storage program area can store the operating system, at least one application program required by the function (such as sound playback function, image playback function, etc.); and the storage data area can store the data created according to the use of the mobile phone (such as audio data, video data, etc.). In addition, the memory may comprise high-speed random-access memories, and further comprise nonvolatile memories, such as hard disks, memories, plug-in hard disks, smart media cards (SMC), secure digital (SD) cards, flash cards (Flash Card), at least one disk memory device, flash devices, or other volatile solid-status memory devices.

Those skilled in the art should understand that the embodiments of the present invention may be provided as methods, systems, servers or computer program products. Therefore, the present invention may adopt the form of a complete hardware embodiment, a complete software embodiment, or an embodiment combining software and hardware. Furthermore, the present invention may be in the form of a computer program product implemented in one or more computer available storage media (comprising but not limited to disk memory and optical memory, etc.) with computer available program codes.

The present invention is described with reference to flowcharts and/or block diagrams of methods, devices (systems), servers and computer program products in embodiments of the present invention. It should be understood that each process and/or block in the flowchart and/or block diagram, and the combination of processes and/or blocks in the flowchart and/or block diagram can be implemented by computer program instructions. These computer program instructions can be provided to the processor of a general-purpose computer, a special-purpose computer, an embedded processor, or other programmable data processing equipment to generate a machine, so that the instructions executed by the processor of the computer or other programmable data processing equipment are caused to generate a device for implementing the functions specified in one or more processes in the flowchart and/or one or more blocks in the block diagram.

These computer program instructions can also be stored in a computer readable memory capable of directing a computer or other programmable data processing devices to work in a particular manner, so that the instructions stored in the computer readable memory produce an article of manufacture comprising a command device. The command device implements the functions specified in one or more processes in the flow diagram and/or one or more blocks in the block diagram.

These computer program instructions may also be loaded onto a computer or other programmable data processing device, so that a series of steps are executed on the computer or other programmable equipment to produce computer-implemented processing, thus, the instructions executed on the computer or other programmable equipment provide steps for implementing the functions specified in one or more processes in the flow chart and/or one or more blocks in the block diagram.

## Claims

1. A computer-implemented, high-precision continuous and rapid detection method for subgrade humidity without damaging a pavement, comprising the following steps:
S1: obtaining ground penetrating radar device GPR map data and capacitively coupled resistivity meter CCR map data of a road site to be detected;
S2: constructing a pavement material dielectric constant-moisture content relationship model, a pavement material resistivity-moisture content relationship model, and a subgrade filling resistivity-moisture content relationship model;
S3: according to the obtained GPR map data, obtaining a pavement layer thickness and a dielectric constant, and according to the obtained pavement layer thickness and the dielectric constant, as well as the pavement material dielectric constant-moisture content relationship model, obtaining a pavement moisture content; and
S4: according to the CCR map data, the pavement thickness and the pavement moisture content, as well as the subgrade filling resistivity-moisture content relationship model, obtaining a subgrade moisture content spatial distribution;
wherein the S4 specifically comprises the following steps:
S41: according to the obtained CCR map data, the pavement layer thickness and the pavement moisture content, obtaining a resistivity spatial distribution within a subgrade range; and
S42: according to the obtained resistivity and the subgrade filling resistivity-moisture content relationship model, obtaining the subgrade moisture content spatial distribution;
wherein the S41 specifically comprises the following steps:
S410: preprocessing the obtained CCR map data, taking the obtained pavement layer thickness and the pavement moisture content as constraints, taking a least squares of the processed CCR map data and forward calculation results of model parameters, and a sum of regularization terms that stabilize inversion results as objective functions, adopting a Gauss-Newton iteration method to solve the solution and obtaining the resistivity spatial distribution within the subgrade range.

2. The computer-implemented, high-precision continuous and rapid detection method for subgrade humidity without damaging the pavement according to claim 1, wherein the S2 specifically comprises the following steps:
S21: according to data, collecting or gathering representative pavement materials and subgrade filling samples to perform dielectric constant and resistivity tests and obtaining test results; and
S22: fitting the test results to obtain the pavement material dielectric constant-moisture content relationship model, the pavement material resistivity-moisture content relationship model, and the subgrade filling resistivity-moisture content relationship model.

3. The computer-implemented, high-precision continuous and rapid detection method for subgrade humidity without damaging the pavement according to claim 1 or claim 2, wherein the S3 specifically comprises the following steps:
S31: preprocessing the obtained GPR map data;
S32: identifying the preprocessed GPR map data and obtaining a pavement structure interface of the road to be detected;
S33: selecting propagation times of radar waves reflected from a bottom of the pavement at different data collection positions on the obtained pavement structure interface;
S34: performing inversion calculations according to a plurality of the obtained propagation times, and obtaining the pavement layer thickness and the dielectric constant; and
S35: according to the obtained dielectric constant and the pavement material dielectric constant-moisture content relationship model, obtaining the pavement moisture content.

4. A high-precision continuous and rapid detection system for subgrade humidity without damaging a pavement, comprising:
an imaging data acquisition module (100), which is configured to obtain ground penetrating radar device GPR map data and capacitively coupled resistivity meter CCR map data of a road site to be detected;
a moisture content relationship model construction module (200), which is configured to construct a pavement material dielectric constant-moisture content relationship model, a pavement material resistivity-moisture content relationship model, and a subgrade filling resistivity-moisture content relationship model;
a pavement moisture content acquisition module (300), which is connected to the imaging data acquisition module and the moisture content relationship model construction module in a communication manner, and is configured to obtain a pavement layer thickness and a dielectric constant according to the obtained GPR map data, and obtain a pavement moisture content according to the obtained pavement layer thickness and the dielectric constant, as well as the pavement material dielectric constant-moisture content relationship model; and
a subgrade moisture content acquisition module (400), which is connected to the imaging data acquisition module, the pavement moisture content acquisition module and the moisture content relationship model construction module in a communication manner, and is configured to obtain a subgrade moisture content spatial distribution according to the CCR map data, the pavement thickness and the pavement moisture content, as well as the subgrade filling resistivity-moisture content relationship model;
wherein the subgrade moisture content acquisition module comprises:
a resistivity acquisition submodule, which is connected to the imaging data acquisition module and the pavement parameter acquisition submodule in a communication manner, and is configured to obtain a resistivity within a subgrade range according to the CCR map data, the pavement layer thickness and the pavement moisture content; and
a submodule moisture content acquisition submodule, which is connected to the resistivity acquisition submodule and the moisture content relationship model construction module in a communication manner, and is configured to obtain the subgrade moisture content spatial distribution according to the obtained resistivity and the subgrade filling resistivity-moisture content relationship model;
the resistivity acquisition submodule is specifically configured to: preprocess the obtained CCR map data, take the obtained pavement layer thickness and the pavement moisture content as constraints, take a least squares of the processed CCR map data and forward calculation results of model parameters, and a sum of regularization terms that stabilize inversion results as objective functions, adopt a Gauss-Newton iteration method to solve the solution and obtain the resistivity spatial distribution within the subgrade range.

5. The high-precision continuous and rapid detection system for subgrade humidity without damaging the pavement according to claim 4, wherein the imaging data acquisition module comprises a multi-channel ground penetrating radar device, a capacitively coupled resistivity meter and a traction vehicle, and the traction vehicle is configured to carry the multi-channel ground penetrating radar device and the capacitively coupled resistivity meter to the road to be detected for data collection.

6. The high-precision continuous and rapid detection system for subgrade humidity without damaging the pavement according to claim 4, wherein the moisture content relationship model construction module comprises:
a test result acquisition submodule, which is configured to collect or gather representative pavement materials and subgrade filling samples according to data to perform dielectric constant and resistivity tests and obtain test results; and
a moisture content relationship model acquisition submodule, which is connected to the test result acquisition submodule in a communication manner, and is configured to fit the test results to obtain the pavement material dielectric constant-moisture content relationship model, the pavement material resistivity-moisture content relationship model, and the subgrade filling resistivity-moisture content relationship model.

7. The high-precision continuous and rapid detection system for subgrade humidity without damaging the pavement according to claim 4, wherein the pavement moisture content acquisition module comprises:
a GPR map data preprocessing submodule, which is connected to the imaging data acquisition module in a communication manner, and is configured to preprocess the obtained GPR map data;
a pavement structure interface acquisition submodule, which is connected to the GPR map data preprocessing submodule in a communication manner, and is configured to identify the preprocessed GPR map data and obtain a pavement structure interface of the road to be detected;
a propagation time acquisition submodule, which is connected to the pavement structure interface acquisition submodule in a communication manner, and is configured to select propagation times of radar waves reflected from a bottom of the pavement at different data collection positions on the obtained pavement structure interface;
a pavement parameter acquisition submodule, which is connected to the propagation time acquisition submodule in a communication manner, and is configured to perform inversion calculations according to a plurality of the obtained propagation times, and obtain the pavement layer thickness and the dielectric constant; and
a pavement moisture content acquisition submodule, which is connected to the pavement parameter acquisition submodule and the imaging data acquisition module in a communication manner, and is configured to obtain the pavement moisture content according to the obtained dielectric constant and the pavement material dielectric constant-moisture content relationship model.

## Patentansprüche

1. Ein computerimplementiertes, hochpräzises, kontinuierliches und schnelles Erfassungsverfahren für Unterbaufeuchtigkeit ohne Beschädigung einer Fahrbahndecke, umfassend die folgenden Schritte:
S1: Erhalten von Bodenradargerät-GPR-Kartendaten und kapazitiv gekoppelten Widerstandsmessgerät-CCR-Kartendaten eines zu erfassenden Straßenstandorts;
S2: Konstruieren eines Fahrbahndeckenmaterial-Dielektrizitätskonstante-Feuchtigkeitsgehalt-Beziehungsmodells, eines Fahrbahndeckenmaterial-Widerstands-Feuchtigkeitsgehalt-Beziehungsmodells und eines Unterbaufüllmaterial-Widerstands-Feuchtigkeitsgehalt-Beziehungsmodells;
S3: gemäß den erhaltenen GPR-Kartendaten, Erhalten einer Fahrbahndeckenschichtdicke und einer Dielektrizitätskonstante, und gemäß der erhaltenen Fahrbahndeckenschichtdicke und der Dielektrizitätskonstante sowie dem Fahrbahndeckenmaterial-Dielektrizitätskonstante-Feuchtigkeitsgehalt-Beziehungsmodell, Erhalten eines Fahrbahndeckenfeuchtigkeitsgehalts; und
S4: gemäß den CCR-Kartendaten, der Fahrbahndeckendicke und dem Fahrbahndeckenfeuchtigkeitsgehalt sowie dem Unterbaufüllmaterial-Widerstands-Feuchtigkeitsgehalt-Beziehungsmodell, Erhalten einer räumlichen Verteilung des Unterbaufeuchtigkeitsgehalts;
wobei S4 spezifisch die folgenden Schritte umfasst:
S41: gemäß den erhaltenen CCR-Kartendaten, der Fahrbahndeckenschichtdicke und dem Fahrbahndeckenfeuchtigkeitsgehalt, Erhalten einer räumlichen Widerstandsverteilung innerhalb eines Unterbaubereichs; und
S42: gemäß dem erhaltenen Widerstand und dem Unterbaufüllmaterial-Widerstands-Feuchtigkeitsgehalt-Beziehungsmodell, Erhalten der räumlichen Verteilung des Unterbaufeuchtigkeitsgehalts;
wobei S41 spezifisch die folgenden Schritte umfasst:
S410: Vorverarbeiten der erhaltenen CCR-Kartendaten, Nehmen der erhaltenen Fahrbahndeckenschichtdicke und des Fahrbahndeckenfeuchtigkeitsgehalts als Randbedingungen, Nehmen von kleinsten Quadraten der verarbeiteten CCR-Kartendaten und Vorwärtsberechnungsergebnissen von Modellparametern sowie einer Summe von Regularisierungstermen, die Inversionsergebnisse stabilisieren, als Zielfunktionen, Anwenden eines Gauß-Newton-Iterationsverfahrens zum Lösen der Lösung und Erhalten der räumlichen Widerstandsverteilung innerhalb des Unterbaubereichs.

2. Das computerimplementierte, hochpräzise, kontinuierliche und schnelle Erfassungsverfahren für Unterbaufeuchtigkeit ohne Beschädigung der Fahrbahndecke nach Anspruch 1, wobei S2 spezifisch die folgenden Schritte umfasst:
S21: gemäß Daten, Sammeln oder Zusammentragen repräsentativer Fahrbahndeckenmaterialien und Unterbaufüllmaterialproben zum Durchführen von Dielektrizitätskonstanten- und Widerstandstests und Erhalten von Testergebnissen; und
S22: Anpassen der Testergebnisse zum Erhalten des Fahrbahndeckenmaterial-Dielektrizitätskonstante-Feuchtigkeitsgehalt-Beziehungsmodells, des Fahrbahndeckenmaterial-Widerstands-Feuchtigkeitsgehalt-Beziehungsmodells und des Unterbaufüllmaterial-Widerstands-Feuchtigkeitsgehalt-Beziehungsmodells.

3. Das computerimplementierte, hochpräzise, kontinuierliche und schnelle Erfassungsverfahren für Unterbaufeuchtigkeit ohne Beschädigung der Fahrbahndecke nach Anspruch 1 oder Anspruch 2, wobei S3 spezifisch die folgenden Schritte umfasst:
S31: Vorverarbeiten der erhaltenen GPR-Kartendaten;
S32: Identifizieren der vorverarbeiteten GPR-Kartendaten und Erhalten einer Fahrbahndeckenstrukturschnittstelle der zu erfassenden Straße;
S33: Auswählen von Laufzeiten von Radarwellen, die von einer Unterseite der Fahrbahndecke an verschiedenen Datenerfassungspositionen auf der erhaltenen Fahrbahndeckenstrukturschnittstelle reflektiert werden;
S34: Durchführen von Inversionsberechnungen gemäß einer Vielzahl der erhaltenen Laufzeiten und Erhalten der Fahrbahndeckenschichtdicke und der Dielektrizitätskonstante; und
S35: gemäß der erhaltenen Dielektrizitätskonstante und dem Fahrbahndeckenmaterial-Dielektrizitätskonstante-Feuchtigkeitsgehalt-Beziehungsmodell, Erhalten des Fahrbahndeckenfeuchtigkeitsgehalts.

4. Ein hochpräzises, kontinuierliches und schnelles Erfassungssystem für Unterbaufeuchtigkeit ohne Beschädigung einer Fahrbahndecke, umfassend:
ein Bildgebungsdatenerfassungsmodul (100), das dazu eingerichtet ist, Bodenradargerät-GPR-Kartendaten und kapazitiv gekoppelte Widerstandsmessgerät-CCR-Kartendaten eines zu erfassenden Straßenstandorts zu erhalten;
ein Feuchtigkeitsgehalt-Beziehungsmodell-Konstruktionsmodul (200), das dazu eingerichtet ist, ein Fahrbahndeckenmaterial-Dielektrizitätskonstante-Feuchtigkeitsgehalt-Beziehungsmodell, ein Fahrbahndeckenmaterial-Widerstands-Feuchtigkeitsgehalt-Beziehungsmodell und ein Unterbaufüllmaterial-Widerstands-Feuchtigkeitsgehalt-Beziehungsmodell zu konstruieren;
ein Fahrbahndeckenfeuchtigkeitsgehalt-Erfassungsmodul (300), das mit dem Bildgebungsdatenerfassungsmodul und dem Feuchtigkeitsgehalt-Beziehungsmodell-Konstruktionsmodul kommunikativ verbunden ist und dazu eingerichtet ist, eine Fahrbahndeckenschichtdicke und eine Dielektrizitätskonstante gemäß den erhaltenen GPR-Kartendaten zu erhalten und einen Fahrbahndeckenfeuchtigkeitsgehalt gemäß der erhaltenen Fahrbahndeckenschichtdicke und der Dielektrizitätskonstante sowie dem Fahrbahndeckenmaterial-Dielektrizitätskonstante-Feuchtigkeitsgehalt-Beziehungsmodell zu erhalten; und
ein Unterbaufeuchtigkeitsgehalt-Erfassungsmodul (400), das mit dem Bildgebungsdatenerfassungsmodul, dem Fahrbahndeckenfeuchtigkeitsgehalt-Erfassungsmodul und dem Feuchtigkeitsgehalt-Beziehungsmodell-Konstruktionsmodul kommunikativ verbunden ist und dazu eingerichtet ist, eine räumliche Verteilung des Unterbaufeuchtigkeitsgehalts gemäß den CCR-Kartendaten, der Fahrbahndeckendicke und dem Fahrbahndeckenfeuchtigkeitsgehalt sowie dem Unterbaufüllmaterial-Widerstands-Feuchtigkeitsgehalt-Beziehungsmodell zu erhalten;
wobei das Unterbaufeuchtigkeitsgehalt-Erfassungsmodul umfasst:
ein Widerstandserfassungs-Untermodul, das mit dem Bildgebungsdatenerfassungsmodul und dem Fahrbahndeckenparameter-Erfassungs-Untermodul kommunikativ verbunden ist und dazu eingerichtet ist, einen Widerstand innerhalb eines Unterbaubereichs gemäß den CCR-Kartendaten, der Fahrbahndeckenschichtdicke und dem Fahrbahndeckenfeuchtigkeitsgehalt zu erhalten; und
ein Untermodul-Feuchtigkeitsgehalt-Erfassungs-Untermodul, das mit dem Widerstandserfassungs-Untermodul und dem Feuchtigkeitsgehalt-Beziehungsmodell-Konstruktionsmodul kommunikativ verbunden ist und dazu eingerichtet ist, die räumliche Verteilung des Unterbaufeuchtigkeitsgehalts gemäß dem erhaltenen Widerstand und dem Unterbaufüllmaterial-Widerstands-Feuchtigkeitsgehalt-Beziehungsmodell zu erhalten;
wobei das Widerstandserfassungs-Untermodul spezifisch konfiguriert ist zum: Vorverarbeiten der erhaltenen CCR-Kartendaten, Nehmen der erhaltenen Fahrbahndeckenschichtdicke und des Fahrbahndeckenfeuchtigkeitsgehalts als Randbedingungen, Nehmen von kleinsten Quadraten der verarbeiteten CCR-Kartendaten und Vorwärtsberechnungsergebnissen von Modellparametern sowie einer Summe von Regularisierungstermen, die Inversionsergebnisse stabilisieren, als Zielfunktionen, Anwenden eines Gauß-Newton-Iterationsverfahrens zum Lösen der Lösung und Erhalten der räumlichen Widerstandsverteilung innerhalb des Unterbaubereichs.

5. Das hochpräzise, kontinuierliche und schnelle Erfassungssystem für Unterbaufeuchtigkeit ohne Beschädigung der Fahrbahndecke nach Anspruch 4, wobei das Bildgebungsdatenerfassungsmodul ein Mehrkanal-Bodenradargerät, ein kapazitiv gekoppeltes Widerstandsmessgerät und ein Zugfahrzeug umfasst, und wobei das Zugfahrzeug dazu eingerichtet ist, das Mehrkanal-Bodenradargerät und das kapazitiv gekoppelte Widerstandsmessgerät für eine Datenerfassung zu der zu erfassenden Straße zu transportieren.

6. Das hochpräzise, kontinuierliche und schnelle Erfassungssystem für Unterbaufeuchtigkeit ohne Beschädigung der Fahrbahndecke nach Anspruch 4, wobei das Feuchtigkeitsgehalt-Beziehungsmodell-Konstruktionsmodul umfasst:
ein Testergebnis-Erfassungs-Untermodul, das dazu eingerichtet ist, repräsentative Fahrbahndeckenmaterialien und Unterbaufüllmaterialproben gemäß Daten zu sammeln oder zusammenzutragen, um Dielektrizitätskonstanten- und Widerstandstests durchzuführen und Testergebnisse zu erhalten; und
ein Feuchtigkeitsgehalt-Beziehungsmodell-Erfassungs-Untermodul, das mit dem Testergebnis-Erfassungs-Untermodul kommunikativ verbunden ist und dazu eingerichtet ist, die Testergebnisse anzupassen, um das Fahrbahndeckenmaterial-Dielektrizitätskonstante-Feuchtigkeitsgehalt-Beziehungsmodell, das Fahrbahndeckenmaterial-Widerstands-Feuchtigkeitsgehalt-Beziehungsmodell und das Unterbaufüllmaterial-Widerstands-Feuchtigkeitsgehalt-Beziehungsmodell zu erhalten.

7. Das hochpräzise, kontinuierliche und schnelle Erfassungssystem für Unterbaufeuchtigkeit ohne Beschädigung der Fahrbahndecke nach Anspruch 4, wobei das Fahrbahndeckenfeuchtigkeitsgehalt-Erfassungsmodul umfasst:
ein GPR-Kartendaten-Vorverarbeitungs-Untermodul, das mit dem Bildgebungsdatenerfassungsmodul kommunikativ verbunden ist und dazu eingerichtet ist, die erhaltenen GPR-Kartendaten vorzuverarbeiten;
ein Fahrbahndeckenstrukturschnittstellen-Erfassungs-Untermodul, das mit dem GPR-Kartendaten-Vorverarbeitungs-Untermodul kommunikativ verbunden ist und dazu eingerichtet ist, die vorverarbeiteten GPR-Kartendaten zu identifizieren und eine Fahrbahndeckenstrukturschnittstelle der zu erfassenden Straße zu erhalten;
ein Laufzeit-Erfassungs-Untermodul, das mit dem Fahrbahndeckenstrukturschnittstellen-Erfassungs-Untermodul kommunikativ verbunden ist und dazu eingerichtet ist, Laufzeiten von Radarwellen auszuwählen, die von einer Unterseite der Fahrbahndecke an verschiedenen Datenerfassungspositionen auf der erhaltenen Fahrbahndeckenstrukturschnittstelle reflektiert werden;
ein Fahrbahndeckenparameter-Erfassungs-Untermodul, das mit dem Laufzeit-Erfassungs-Untermodul kommunikativ verbunden ist und dazu eingerichtet ist, Inversionsberechnungen gemäß einer Vielzahl der erhaltenen Laufzeiten durchzuführen und die Fahrbahndeckenschichtdicke und die Dielektrizitätskonstante zu erhalten; und
ein Fahrbahndeckenfeuchtigkeitsgehalt-Erfassungs-Untermodul, das mit dem Fahrbahndeckenparameter-Erfassungs-Untermodul und dem Bildgebungsdatenerfassungsmodul kommunikativ verbunden ist und dazu eingerichtet ist, den Fahrbahndeckenfeuchtigkeitsgehalt gemäß der erhaltenen Dielektrizitätskonstante und dem Fahrbahndeckenmaterial-Dielektrizitätskonstante-Feuchtigkeitsgehalt-Beziehungsmodell zu erhalten.

## Revendications

1. Procédé de détection continue et rapide, de haute précision, mis en œuvre par ordinateur, de l'humidité de la plate-forme de terrassement sans endommager un revêtement routier, comprenant les étapes suivantes:
S1: l'obtention de données cartographiques issues d'un radar à pénétration de sol (GPR) et de données cartopgraphiques d'un résistivimètre à couplage capacitif (CCR) pour un site routier à analyser;
S2: élaboration d'un modèle de relation entre la constante diélectrique et la teneur en humidité du matériau du revêtement routier, d'un modèle de relation entre la résistivité et la teneur en humidité du matériau du revêtement routier, et d'un modèle de relation entre la résistivité du remblai de la plate-forme de terrassement et sa teneur en humidité;
S3: à partir des données cartographiques GPR obtenues, déterminer l'épaisseur de la couche de revêtement routier et sa constante diélectrique; puis, à partir de l'épaisseur de la couche de revêtement routier et de la constante diélectrique ainsi obtenues, ainsi que du modèle de relation entre la constante diélectrique et la teneur en humidité du matériau de revetement routier, déterminer la teneur en humidité du revetement routier; et
S4: à partir des données cartographiques CCR, de l'épaisseur du revêtment routier et de la teneur en humidité du revêtement routier, ainsi que du modèle de relation entre la résistivité et la teneur en humidité du sol de fondation, déterminer la répartition spatiale de la teneur en humidité du remblai de la plate-forme de terrassement;
l'étape S4 comprenant plus précisément les étapes suivantes:
S41: à partir des données cartographiques CCR obtenues, de l'épaisseur de la couche du revêtement routier et de la teneur en humidité du revêtement routier, déterminer la répartition spatiale de la résistivité dans une plage de la plate-forme de terrassement; et
S42: à partir de la résistivité obtenue et du modèle de relation entre la résistivité et la teneur en humidité de la plate-forme de terrassement, déterminer la répartition spatiale de la teneur en humidité de la plate-forme de terrassement;
dans lequel l'étape S41 comprend plus précisément les étapes suivantes:
S410: prétraiter les données cartographiques CCR obtenues, en prenant comme contraintes l'épaisseur de la couche de revêtement routier et la teneur en humidité de celle-ci, en prenant comme fonctions objectif la méthode des moindres carrés des données cartographiques CCR traitées et des résultats de calcul direct des paramètres du modèle, ainsi qu'une somme de termes de régularisation qui stabilisent les résultats d'inversion, en adoptant une méthode d'itération de Gauss-Newton pour résoudre l'équation et obtenir la distribution spatiale de la résistivité dans la zone de la couche de fondation.

2. Procédé de détection continue et rapide de haute précision, mis en œuvre par ordinateur, de l'humidité de la plate-forme de terrassement sans endommager le revêtement routier selon la revendication 1, dans lequel l'étape S2 comprend spécifiquement les étapes suivantes:
S21: en fonction des données, collecter ou prélever des échantillons représentatifs de matériaux de revêtement routier et du remblai de la plate-forme de terrassement afin d'effectuer des essais de constante diélectrique et de résistivité et d'obtenir les résultats des essais; et
S22: ajuster les résultats des essais afin d'obtenir le modèle de relation entre la constante diélectrique et la teneur en humidité des matériaux de revêtement routier, le modèle de relation entre la résistivité et la teneur en humidité des matériaux de revêtement routier, et le modèle de relation entre la résistivité et la teneur en humidité du remblai de la plate-forme de terrassement.

3. Procédé de détection continue et rapide, de haute précision, mis en œuvre par ordinateur, de l'humidité de la plate-forme de terrassement sans endommager le revêtement routier, selon la revendication 1 ou la revendication 2, dans lequel l'étape S3 comprend spécifiquement les étapes suivantes:
S31: prétraitement des données cartographiques GPR obtenues;
S32: identification des données cartographiques GPR prétraitées et obtention d'une interface de structure du revêtement routier de la route à détecter;
S33: sélectionner les temps de propagation des ondes radar réfléchies par la face inférieure du revêtement routier à différentes positions de collecte de données sur l'interface de structure du revêtement routier obtenue;
S34: effectuer des calculs d'inversion en fonction d'une pluralité des temps de propagation obtenus, et déterminer l'épaisseur de la couche du revêtement routier et la constante diélectrique; et
S35: en fonction de la constante diélectrique obtenue et du modèle de relation entre la constante diélectrique et la teneur en humidité du matériau du revêtement routier, obtenir la teneur en humidité du revêtement routier.

4. Système de détection continue et rapide de haute précision de l'humidité de la plate-forme de terrassement sans endommager le revêtement routier, comprenant:
un module d'acquisition de données d'imagerie (100), qui est configuré pour obtenir des données cartographiques provenant d'un radar à pénétration de sol (GPR) et des données cartographiques provenant d'un résistivimètre à couplage capacitif (CCR) d'un site routier à détecter;
un module de construction de modèles de relation entre la teneur en humidité (200), configuré pour construire un modèle de relation entre la constante diélectrique du matériau du revêtement routier et la teneur en humidité, un modèle de relation entre la résistivité du matériau du revêtement routier et la teneur en humidité, et un modèle de relation entre la résistivité du remblai de la plate-forme de terrassement et la teneur en humidité;
un module d'acquisition de la teneur en humidité du revêtement routier (300), qui est relié de manière communicante au module d'acquisition de données d'imagerie et au module de construction de modèles de relation de teneur en humidité, et qui est configuré pour déterminer l'épaisseur de la couche du revêtement routier et la constante diélectrique à partir des données cartographiques GPR obtenues, ainsi que pour déterminer la teneur en humidité du revêtement routier à partir de l'épaisseur de la couche du revêtement routier et de la constante diélectrique obtenues, ainsi que du modèle de relation entre la constante diélectrique et la teneur en humidité du matériau du revêtement routier; et
un module d'acquisition de la teneur en humidité de la plate-forme de terrassement (400), qui est relié de manière communicante au module d'acquisition de données d'imagerie, au module d'acquisition de la teneur en humidité du revêtement routier et au module de construction du modèle de relation entre la constante diélectrique et la teneur en humidité, et qui est configuré pour déterminer une distribution spatiale de la teneur en humidité de la plate-forme de terrassement en fonction des données cartographiques CCR, de l'épaisseur du revêtement routier et de la teneur en humidité du revêtement routier , ainsi que le modèle de relation entre la résistivité du remblai de la plate-forme de terrassement et la teneur en humidité;
dans lequel le module d'acquisition de la teneur en humidité de la plate-forme de terrassement comprend:
un sous-module d'acquisition de la résistivité, qui est relié de manière communicante au module d'acquisition des données d'imagerie et au sous-module d'acquisition des paramètres du revêtement routier, et qui est configuré pour déterminer une résistivité dans une plage de la couche de fondation en fonction des données cartographiques CCR, de l'épaisseur de la couche de revêtement routier et de la teneur en humidité du revêtement routier; et
un sous-module d'acquisition de la teneur en humidité de la plate-forme de terrassement, qui est relié de manière communicante au sous-module d'acquisition de la résistivité et au module de construction du modèle de relation entre la résistivité et la teneur en humidité de la plate-forme de terrassement, et qui est configuré pour obtenir la distribution spatiale de la teneur en humidité de la plate-forme de terrassement en fonction de la résistivité obtenue et du modèle de relation entre la résistivité et la teneur en humidité de la plate-forme de terrassement;
le sous-module d'acquisition de la résistivité est spécifiquement configuré pour: prétraiter les données de la carte CCR obtenues, prendre comme contraintes l'épaisseur de la couche dur revêtement routier et la teneur en humidité du revêtement routier, utiliser comme fonctions objectif la méthode des moindres carrés appliquée aux données traitées de la carte CCR et les résultats de calcul des paramètres du modèle, ainsi qu'une somme de termes de régularisation qui stabilisent les résultats de l'inversion, et adopter une méthode d'itération de Gauss-Newton pour résoudre l'équation et obtenir la distribution spatiale de la résistivité au sein de la plate-forme de terrassement.

5. Système de détection continue et rapide de haute précision de l'humidité de la plate-forme de terrassement sans endommager le revêtement routier, selon la revendication 4, dans lequel le module d'acquisition de données d'imagerie comprend un dispositif radar à pénétration de sol multicanaux, un résistivimètre à couplage capacitif et un véhicule de traction, et le véhicule de traction est configuré pour transporter le dispositif radar à pénétration de sol multicanaux et le résistivimètre à couplage capacitif jusqu'à la route à détecter en vue de la collecte de données.

6. Système de détection continue et rapide de haute précision de l'humidité de la plate-forme de terrassement sans endommager le revêtement routier, selon la revendication 4, dans lequel le module de construction du modèle de relation de la teneur en humidité comprend:
un sous-module d'acquisition des résultats d'essai, qui est configuré pour collecter ou prélever des échantillons représentatifs des matériaux de revêtement et du remblai de la plate-forme de terrassement en fonction des données, afin d'effectuer des essais de constante diélectrique et de résistivité et d'obtenir des résultats d'essai; et
un sous-module d'acquisition de modèles de relation de teneur en humidité, qui est relié au sous-module d'acquisition des résultats d'essai par une liaison de communication, et qui est configuré pour ajuster les résultats d'essai afin d'obtenir le modèle de relation entre la constante diélectrique et la teneur en humidité des matériaux du revêtement routier, le modèle de relation entre la résistivité et la teneur en humidité des matériaux du revêtement routier, et le modèle de relation entre la résistivité et la teneur en humidité du remblai de la plate-forme de terrassement.

7. Système de détection continue et rapide de haute précision de l'humidité de la plate-forme de terrassement sans endommager le revêtement routier, selon la revendication 4, dans lequel le module d'acquisition de la teneur en humidité du revêtement routier comprend:
un sous-module de prétraitement des données cartographiques GPR, qui est relié au module d'acquisition des données d'imagerie de manière communicante, et qui est configuré pour prétraiter les données cartographiques GPR obtenues;
un sous-module d'acquisition de l'interface de la structure du revêtement routier, qui est relié au sous-module de prétraitement des données cartographiques GPRde manière communicante, et qui est configuré pour identifier les données cartographiques GPR prétraitées et obtenir une interface de la structure du revêtement routier de la route à détecter;
un sous-module d'acquisition des temps de propagation, qui est relié au sous-module d'acquisition de l'interface de structure du revêtement routier de manière communicante, et qui est configuré pour sélectionner les temps de propagation des ondes radar réfléchies par la face inférieure du revêtement routier à différentes positions de collecte de données sur l'interface de structure du revêtement routier obtenue;
un sous-module d'acquisition des paramètres du revêtement routier, qui est relié au sous-module d'acquisition des temps de propagationde manière communicante, et qui est configuré pour effectuer des calculs d'inversion en fonction d'une pluralité des temps de propagation obtenus, et pour déterminer l'épaisseur des couches du revêtement routier et la constante diélectrique; et
un sous-module d'acquisition de la teneur en humidité du revêtement routier, qui est relié de manière communicante, au sous-module d'acquisition des paramètres du revêtement routier et au module d'acquisition des données d'imagerie, et qui est configuré pour déterminer la teneur en humidité du revêtement routier en fonction de la constante diélectrique obtenue et du modèle de relation entre la constante diélectrique et la teneur en humidité du matériau du revêtement routier.
